# EUROPEAN PATENT SPECIFICATION

(11) **EP 4 307 913 B1**
(45) Date of publication and mention of the grant of the patent: **30.07.2025**
(21) Application number: 22725229.3
(22) Date of filing: 25.04.2022
(51) Int. Cl.: A23J 3/20, A23J 3/22, A23L 31/00, A23L 33/195, C12M 1/16, C12N 1/14, A23J 3/26

(54) **A METHOD FOR MANUFACTURING A PROTEIN-RICH BIOMASS COMPRISING EDIBLE FILAMENTOUS FUNGUS**
VERFAHREN ZUR HERSTELLUNG EINER PROTEINREICHEN BIOMASSE MIT EINEM ESSBAREN FILAMENTÖSEN PILZ
PROCÉDÉ DE FABRICATION D'UNE BIOMASSE RICHE EN PROTÉINES COMPRENANT DES CHAMPIGNONS FILAMENTEUX COMESTIBLES

(30) Priority: 30.04.2021 EP 21171629
(43) Date of publication of application: 24.01.2024
(73) Proprietor: Millow Holding AB, 421 30 Västra Frölunda (SE)
(72) Inventor: TAHERZADEH, Mohammad J., Hisings Backa (SE); HELLWIG, Coralie, Göteborg (SE); LUKITAWESA, Lukitawesa, Göteborg (SE); WAINAINA, Steven, Borås (SE); AGNIHOTRI, Swarnima, Landvetter (SE)
(74) Representative: AWA Sweden AB
(86) International application number: PCT/EP2022/060852
(87) International publication number: WO 2022/229072

(56) References cited:
- EP-A1- 2 835 058
- WO-A1-2020/232347
- US-A1- 2014 342 036
- US-A1- 2015 166 945
- W. GRAJEK: "Production of protein by thermophilic fungi from sugar-beet pulp in solid-state fermentation", BIOTECHNOLOGY AND BIOENGINEERING, vol. 32, no. 2, 5 July 1988 (1988-07-05), US, pages 255 - 260, XP055359736, ISSN: 0006-3592, DOI: 10.1002/bit.260320218
- K H STEINKRAW: "Solid-state (Solid-Substrate) Food/Beverage Fermentations Involving Fungi", ACTA BIOTEHNOLOGICY, 1 January 1984 (1984-01-01), pages 83 - 88, XP055115684, Retrieved from the Internet <URL:http://onlinelibrary.wiley.com/store/10.1002/abio.370040202/asset/370040202_ftp.pdf?v=1&t=hukwgh22&s=36acbb9b16986cb8b34a7541cad4134d06afd7cf> [retrieved on 20140429]

## Description

### TECHNICAL FIELD

The present invention relates to a method for manufacturing a protein-rich biomass comprising at least one fermented substrate and at least one strain of an edible filamentous fungus.

### BACKGROUND OF THE INVENTION

There has been a growing awareness in the population regarding proper nutrition habits, in particular considering global climate change situation, which has resulted in an ever-increasing interest in developing functional food products being beneficial from both health and environmental aspects. Production of both probiotic and prebiotic products has catapulted due to the presence of certain immune boosting/therapeutic compounds in these products. Further, such functional food products are protein-rich, which makes these products highly suitable as plant-based meat replacement.

Although consumption of protein from animal sources has been increasing in developed countries over the last few decades, around one billion people in the world currently do not have access to a diet which provides enough protein and energy. Lack of protein can result in severe health problems such as growth failure, muscle weakness and impaired immune system.

The production of meat has a heavy impact on the environment and makes a large contribution to the eutrophication process. Therefore, it is desirable to find an alternative, cheap, and less resource-consuming protein source to substitute meat or meat products.

US2014/0342036 discloses a vegetable-based food product comprising at least 50 wt.% protein, wherein the protein is gluten or is proteinaceous material derived from gluten, and wherein the product has the texture of minced meat.

Fungal organisms such as mushrooms and truffles have traditionally been a part of human nutrition. However, they cannot be considered as an important source of protein in comparison to animal-based sources. Considerable attention has recently been given to the use of filamentous fungi as a commercial human food component, especially due to their high protein content and presence of amino acids essential to human nutrition. Food products comprising filamentous fungi are easily digestible, have low fat content, are cholesterol free, and comprise dietary fibers in an amount comparable with other vegetarian protein sources.

Several strains of edible filamentous fungi have been recognized as a traditional source of palatable food by many societies around the globe, especially in Asia [8]. *Rhizopus sp.* has been used for centuries in the oriental cuisine in the preparation of fermented food such as tempeh. *Aspergillus oryzae* also has culinary applications for the production of hamanatto, miso and shoyu. *Neurospora intermedia* is used in the preparation of the Indonesian staple food oncom. Similarly, *Monascus purpureus* has been used as coloring and flavoring agent in food and beverages, as in the production of red yeast rice and rice wine. Other applications of filamentous fungi include production of ingredients for food and beverage industries, such as enzymes. In recent years, production of vitamins and polyunsaturated fatty acids by these microorganisms has been receiving increased attention. Single-cell protein (SCP) can also be produced by filamentous fungi. An example of SCP currently being available in the market is the filamentous fungus *Fusarium venenatum,* commercialized under the name Quorn^{®}, comprising 25% fiber, 13% lipid and 50% protein on dry cell weight basis.

Fungal fermentation products may be produced either by liquid state, or submerged fermentation (SMF), or by solid state fermentation (SSF).

The most common method of fermenting a substrate using edible filamentous fungi is SMF. Mycoprotein is a commonly used term defining protein-rich food made of filamentous fungal biomass and is usually referred to plant-based alternative to meat. Mycoprotein in Quorn^{®} is produced using SMF from the filamentous fungi *Fusarium venenatum.* Further, a SMF process using potato protein liquor has been described, in which a protein-rich biomass was obtained using airlift bioreactor (Souza Filho et al, Fermentation, 2017, 3(1), p. 12). Another example of using an airlift bioreactor is for using byproduct from pea processing that has been converted by several filamentous fungi strains into edible biomass (Souza Filho et al., Fungal Biology and Biotechnology, 2018, 5(1), p. 5).

Despite the fact that SMF is widely used and constantly developed, large-scale production of edible filamentous fungal biomass using SMF is still commercially challenging, primarily due to huge volumes of water required in such a process. Further, SMF is an artificial condition for filamentous fungi because they live in solid state in nature.

In order to remedy the shortcomings of SMF, SSF may be used instead. The process of SSF is normally carried out by growing fungi on the moist water-insoluble solid substrate substantially in the absence of free liquid, thus avoiding excess water and offering the advantage of low energy consumption along with a high product concentration. Since the required amount of liquid is absorbed by the solid substrate, the growth of microorganisms is improved due to unobstructed transfer of oxygen to the microorganisms. Although SMF is sometimes preferred over SSF due to a simpler downstream processing of metabolites, SSF offers a number of significant advantages over SMF, such as a reduced water consumption, possibility to use cellulosic waste as a substrate, as well as small fermentation reactors. Considering the above, SSF is more environmentally benign and economical industrial process as compared to SMF.

SSF is known to introduce the probiotic functionalities to breakfast cereals and similar food products, by cultivating *Lactobacillus plantarum* strain on oat bran and spent oats after lipid extraction and limited hydrolysis of the raw materials by *Aspergillus awamori* and *Aspergillus oryzae.* Further, a study performed in an Erlenmeyer flask using various fungi such as *Aspergillus oryzae* concluded that fermented oat had superior antioxidant properties relative to the unfermented ones (Cai, S.,et al., Journal of Agricultural and Food Chemistry, 2012. 60(1), p. 507-513). The mycoprotein from brand Quorn^{®} mentioned above has been reported to have numerous nutritional benefits which is obtained by growing a filamentous fungus under liquid state fermentation. Hyphal cell-walls contain chitin, 1,3- and 1,6-glucan and are a source of dietary fiber. The fiber content itself may work as a prebiotic in gut, cell membranes are rich in polyunsaturated fats and cytoplasm rich in high quality protein. The amino acids of mycoprotein are composed of all essential ones. Therefore, by fermentation it is possible to improve the nutritional properties of the raw materials.

Despite its benefits, industrial application of SSF is still limited, since the problem of an efficient mass and heat transfer has not yet been solved for a large scale application. In particular, heat dissipation remains a major challenge in SSF since it may be limited by inter- and intra-particle conflicts and is difficult to control. Specific sensors and solid handling techniques are required to overcome this problem and to carry out the fermentation process continuously. Another important parameter that needs to be carefully controlled during SSF is water activity (a_{w}), i.e. the partial vapor pressure of water in a solution divided by the standard state partial vapor pressure of water. An optimal moisture level is very important in order to achieve a proper growth of fungi. Too low moisture level adversely affects fungal growth and tends to cause swelling of substrate, enzyme stability and nutrient diffusion. On the other hand, too high moisture level is associated with bacterial contamination, agglomeration of substrate particles and limited gas transfer. The optimal moisture level varies depending on the type of fungus and the nature of substrate. Most of the fungi capable of growing under SSF conditions have water activity of at least 0.8-0.9. For a particular fungus to grow on a specific substrate, a number of factors should be considered, e.g. particle size and shape, macromolecular structure, porosity, and particle consistency of the substrate. Thus, a larger surface area to volume ratio is required to obtain high yields as it facilitates penetration of the substrate by the fungus such that the substrate eventually becomes more accessible for the hydrolytic enzymes. If the surface area is insufficient, enzyme diffusion becomes the rate limiting step of the SSF.

For a long time in history, SSF had been utilized to produce indigenous food in different parts of the world. The traditional fermentations food products such as Japanese "koji", Indonesian "tempeh", Indian "ragi", and French "blue cheese" can be mentioned as some of the oldest documented applications of SSF. Several substrates have been utilized to produce these traditional fermentation products. For instance, tempeh is usually produced by inoculating boiled and dehulled soyabeans or chickpeas with *Rhizopus oligosporus* spores. Thereafter, fermentation is carried out in warm and humid environments without forced air using polyethylene bags with several holes or a zipper. Indeed, such systems cannot adequately control key fungal growth parameters such as temperature, humidity, or oxygen supply.

Recent research has demonstrated alternative SSF techniques using fungi belonging to *Zygomycota* and *Ascomycota* phyla. For example, breadcrumbs and brewers spent grain may be converted to fungal biomass using laboratory petri dishes (Gmoser, R., et al., Bioengineered, 2020, 11(1), p. 582-598).

Considering the disadvantages of SMF, and also limitations of currently available SSF processes, it is desirable to provide a cost-efficient and environmentally benign method a system for SSF having a production capacity of several tons per day.

### SUMMARY OF THE INVENTION

In view of the above, the present invention thus provides such a method, not only having low water requirement, but also being readily up-scalable.

The present invention is defined by the appended claims. The method for manufacturing a protein-rich biomass comprising at least one fermented substrate and at least one strain of an edible filamentous fungus according to the present invention comprises the steps of:
a) providing at least one substrate to be fermented, the substrate(s) having total solid loading of from 51% to 85%; wherein said at least one substrate is selected from a group consisting of root vegetables and residues and derivatives thereof, grains and residues and derivatives thereof, legumes and residues and derivatives thereof, marine organisms and residues and derivatives thereof, potato protein liquor (PPL), distillers dried grain with solubles (DDGS) as well as precursors and residue thereof, and mixtures thereof;
b) inoculating the at least one substrate with at least one strain of an edible filamentous fungus thus obtaining at least one inoculated substrate;
c) setting a set of conditions, wherein the set of conditions comprises at least one of humidity level of from 20% to 100%, flow rate of a second gaseous fluid at 0.25-4 vvm, temperature of from 30°C to 45°C, light intensity of from 4·10¹⁴ Hz to 5·10¹⁴ Hz and pH of from 3 to 7;
d) fermenting the at least one inoculated substrate at the set of conditions while continuously monitoring at least one parameter, thus obtaining a protein-rich biomass comprising at least one fermented substrate and at least one strain of an edible filamentous fungus;

wherein said at least one strain of an edible filamentous fungus is selected from a group consisting of *Rhizopus* spp., *Aspergillus* spp., *Neurospora* spp., *Monascus* spp. and *Rhizomucor* spp., and
wherein the set of conditions is amended as a function of the value of the at least one parameter.

The term "as a function" in the context of the present invention is intended to mean that a change in the value of the at least one parameter above a predefined threshold triggers an amendment in the set of conditions. In other words, if no change in the value of the at least one parameter is detected, or if the change is below the predefined threshold, the set of conditions will remain unamended.

By the term "edible" in the context of the present invention is meant being suitable for human and/or animal consumption. By the term "animal" is understood species belonging to the fauna, such as vertebrates and invertebrates.

The at least one substrate in step a) is selected from a group consisting of root vegetables and residues and derivatives thereof, grains and residues and derivatives thereof, legumes and residues and derivatives thereof, marine organisms and residues and derivatives thereof, potato protein liquor (PPL), distillers dried grain with solubles (DDGS) as well as precursors and residue thereof, and mixtures thereof. Examples of root vegetables include but are not limited to: potato, cassava, beetroot, carrot, or the like. Examples of grains include but are not limited to: barley, oat, wheat, rye, corn, or the like. Examples of legumes include but are not limited to: soya bean, pea, chickpea, or the like. Examples of marine organisms include but are not limited to: algae, sea squirt or the like.

The substrates mentioned above may be used in combinations to achieve an effective synergistic effect to promote the growth of edible filamentous fungi. The substrates mentioned above may be combined with each other and with at least one strain of edible filamentous fungus in order to achieve the desired functionality in the targeted protein-rich biomass. Using industrial byproducts like PPL and DDGS provides the advantage of contributing to the circular economy.

In particular, the substrate may be DDGS or a mixture of root vegetables and grains. Further, the substrate mixture may be combination of DDGS with soya beans or pea protein. Such a substrate mixture is particularly suitable for producing fish feed. Other preferred combinations of different substrates particularly suitable for manufacturing a protein-rich biomass for human consumption include beetroots and potatoes, oat bran and carrots, and barley and cassava. When a mixture of two substrates is used, the ratio between the substrates may be from 1:1 to 1:16, more preferably from 1:2 to 1:4.

An essential feature of the present invention is the relatively high total solid loading of the substrate of at least 51%. Further, the total solid loading of the substrate should not exceed 85%. As indicated by such a high total solid loading, the method of the present invention is a SSF process, which greatly reduces the utilization of water. The method of the present invention produces no effluent like wastewater for further treatment and hence is an environmentally benign and contributes to the circular economy.

The high total solid loading is preferably achieved by soaking the substrate in liquid for a time period from 10 to 120 min, and then filtering off the excess liquid. Alternatively, the at least one substrate may already contain a sufficient amount of liquid in order to enable growth of the at least edible filamentous fungus.

The method of the present invention may comprise a step a") of pretreating the at least one substrate. If present, step a") occurs before step a). Such a pretreating may be boiling, steaming, soaking, drying, chopping, mixing, crushing, peeling or any combinations thereof.

The method according to the present invention may further comprise step b') of adjusting pH value of the substrate to be from 3 to 7, preferably from 4.8 to 6.8, more preferably from 5.6 to 6.2. If present, step b') occurs before step b). The pH value may be adjusted by addition of suitable acidic agents, such as hydrochloric acid, or alkaline agents, such as sodium hydroxide and/or potassium hydroxide.

Inoculation of the at least one substrate mentioned above with at least one strain of an edible filamentous fungus in step b) may be performed by any suitable method, such as mixing the at least one substrate with the edible filamentous fungus, sprinkling the edible filamentous fungus on top of the substrate, or combination of both of these methods. The ratio between the edible filamentous fungus and the substrate may be from 1:1 to 1:10000, more preferably from 1:35 to 1:50. Once the edible filamentous fungus has been introduced into the substrate, an at least one inoculated substrate is obtained. Controlled inoculation is enabled by keeping the water activity a_{w} of the inoculated substrate at approximately 0.9 and maintaining an acidic microenvironment with a pH range of from 3 to 7.

It should be noted that step d) may be performed under aerobic or anaerobic conditions and may occur within a fermentation reactor.

Under aerobic conditions, in order to achieve an optimal growth of the edible filamentous fungus during fermentation process, the following factors have to be considered: (i) effective supply of oxygen, (ii) sufficient heat transfer and (iii) controlled amount of humidity. Thus, in the next step c), a set of conditions is determined and set, wherein the set of conditions comprises at least one of humidity level, flow rate of a second gaseous fluid, temperature, light intensity and pH as mentioned above. The light intensity may be from 4.5·10¹⁴ Hz to 5·10¹⁴ Hz, more preferably 4.8·10¹⁴ Hz.

The humidity level is within the range from 20 to 100% Rh. When adjusting the humidity level to be within the specified range, it may be necessary to increase humidity level by adding water vapor, or to decrease the humidity level by removing water vapor. Humidification or dehumidification may be performed using any conventional method known in the art. The set of conditions mentioned above may further comprise a flow rate of a second gaseous fluid. The second gaseous fluid may be air, oxygen (O₂) or nitrogen (N₂). In particular, the second gaseous fluid is air. The flow rate of the second gaseous fluid is set to a first value being at least 0.25 vvm (volume gas flow per unit of bed volume per minute), and not more than 4 vvm. Further, the set of conditions may comprise temperature, that is set to be in the mesophilic range of 30°C to 45°C, more preferably from 35°C to 40°C. Preferably, the set of conditions comprises two of humidity level, air flow rate and temperature, more preferably all three of humidity level, air flow rate and temperature within the specified ranges.

During the next step d), the inoculated substrate is fermented starting with the set of conditions obtained in step c) while continuously monitoring at least one parameter, thus obtaining a protein-rich biomass comprising at least one fermented substrate and at least one strain of an edible filamentous fungus. The at least one parameter may be at least one of the level of at least one first gaseous fluid, a temperature, a humidity level, light intensity and pH. Further, the at least one parameter may be any other parameter being relevant for fermentation process. The at least one parameter is preferably measured in proximity of the inoculated substrate. The at least one first gaseous fluid may be carbon dioxide (CO₂), ammonia (NH₃), oxygen (O₂) or nitrogen (N₂). Preferably, the at least one parameter being continuously monitored during step d) is the level of CO₂.

The at least one parameter mentioned above will change depending on the propagation phase of the at least one edible filamentous fungus. According to the present invention, the set of conditions is amended as a function of the at least one parameter. In other words, the method of the present invention is adapted to the propagation phase of the at least one edible filamentous fungus.

In order to achieve optimal growth of the edible filamentous fungus on the substrate to be fermented, thus maximizing the yield, and improving the quality of the protein-rich biomass, different optimal air flow, heat and humidity conditions are needed for different growth stages. For instance, during active growth phase, higher heat and CO₂ dissipation is expected and thus the set of conditions comprising at least one of supplied second gaseous fluid, temperature, humidity level, light intensity and pH will automatically be adjusted to maintain the optimal growth environment according to the present invention. For example, the air flow rate set in step c) may be automatically adjusted as a function of the level of CO₂ monitored in step d). In particular, when the CO₂ levels are in the range of 20000-30000 ppm, the air flow rate set in step d) may automatically be adjusted to a second value, that may be in the range of 0.5-4 vvm. Usually, the second value is greater than the first value.

Step d) may be performed during a time period from 10 h to 80 h, preferably from 24 h to 72 h, more preferably from 40 h to 50 h, depending on the type and amount of the inoculated substrate, the strain of the at least edible filamentous fungus, and the set of conditions defined in step c).

It is conceivable that the time period mentioned above comprises a plurality of time intervals, wherein the set of conditions is different during at least two time intervals. In particular, the temperature may be raised and lowered repeatedly during the time period of step d), e.g. such that the temperature has a first value during the first time interval, a second value during the second interval, a third value during the third time interval and so on. The duration of the time intervals may be same or different.

In order to avoid contamination of the substrate to be fermented, human contact should be minimized, especially during step d). To this end, the method of the present invention may further comprise step c') of robot-assisted loading the at least one inoculated substrate into a fermentation chamber and/or step e) of robot-assisted unloading of the protein-rich biomass from the fermentation chamber. By introduction of these steps, human-induced contamination is greatly reduced or even eliminated since the robots may be exclusively remotely controlled without interaction with the fermentation area. By the term "robot" in the context of the present invention is understood a machine, especially one programmable by a computer, capable of carrying out a complex series of preferably mechanic actions automatically.

In order to improve texture of the protein-rich biomass obtainable by the method of the present invention, the method may further comprise step a') of adding at least one binder, wherein the step a') occurs simultaneously with step a). The binder may be selected from the group consisting of methyl cellulose, potato starch gel, kappa carrageenan, sodium alginate, corn starch and combinations thereof.

Further, the method according to the present invention may comprise step f) of heat treatment of the protein-rich biomass, wherein step f) occurs immediately after step d). This step may be performed in order to reduce the nucleic acid (RNA) content. During this step, the temperature may be raised to 65°C for a period of 20-30 minutes. The RNA degrades into monomers and diffuses out of cells leaving the RNA content of fungus well below 2% by weight.

It may be desirable to preserve the protein-rich biomass for a prolonged period of time, for instance for transportation, or to prepare the protein-rich biomass for a further processing. To this end, the method according to the present invention may further comprise step g) of freezing the protein-rich biomass. If present, step g) is performed before step e), i.e. before unloading the protein-rich biomass from the fermentation chamber. The freezing cycles may be performed by reducing the temperature down to -70°C for up to 2 hours. Preferably, the freezing is conducted at a temperature from -50°C to -40°C for up to 1 hour.

The method of the present invention may comprise step h) of drying the protein rich biomass. Step h) may be performed as follows:
- ramping up the temperature to 40-90°C at a rate of between 5-10°C per minute;
- maintaining the temperature at 40-90°C for 3-8 hours.

It should be noted that step g) and step h) may be performed in any order.

It is also conceivable that the method of the present invention comprises step i) of post-fermentation sterilization procedure, e.g. pasteurization or microwave heating to yield a safe final protein-rich biomass having a long shelf life.

According to the present invention, the at least one strain of an edible filamentous fungus is selected from a group consisting of *Rhizopus* spp., *Aspergillus* spp., *Neurospora* spp., *Monascus* spp. or *Rhizomucor* spp. As mentioned above, it is conceivable to use any combination of these edible filamentous fungi in combination with any substrate or combination of substrates as mentioned above.

As may be understood, the method of the present invention allows for a large scale SSF manufacturing of protein-rich biomass comprising at least one fermented substrate and at least one strain of edible filamentous fungus, resulting in high production capacity of several tons a day. The method of the present invention provides uniform mass transfer of air, distribution of temperature and humidity during propagation of at least one edible filamentous fungus. The method of the present invention may be completely automated, wherein each of the steps described above is performed without human interaction. Finally, the method of the present invention is highly cost efficient since it uses the cheap substrates and negligible amount of water.

A protein-rich biomass is manufactured by the method described above. The protein-rich biomass thus comprises at least one fermented substrate and at least one strain of edible filamentous fungus.

The protein-rich biomass may have a high protein content of at least 10%, preferably at least 20%, more preferably at least 30%. The protein-rich biomass may comprise up to 60% protein.

As mentioned above, the protein-rich biomass obtained by the process of the present invention is highly nutritious and may comprise at least one amino acid, at least one prebiotic substance, at least one probiotic substance, at least one mineral, at least one vitamin, at least one dietary fiber or combinations thereof. The at least one amino acid may be one of the essential amino acids. Preferable, the protein-rich biomass comprises at least five essential amino acids, preferably at least eight essential amino acids. In other words, the protein-rich biomass may have the desirable essential amino acid pattern for human consumption.

The probiotic substance may be good gut bacteria, e.g. *Lactobacilli.* The prebiotic substance may be the dietary fiber present in the fermented product. The vitamin may be vitamin B, in particular vitamin B₂, B₉, B₁₂, choline and combination thereof. The mineral may be zinc, iron, manganese, sodium, phosphorus, and combination thereof.

Disclosed, but not claimed is the protein-rich biomass of the present disclosure, which is normally low in fat and high in polyunsaturated fatty acids like omega-3 and omega-6, and/or high in mobilized phenolic antioxidants. The functionality of the non-claimed protein-rich biomass is increased many-fold in terms of total phenolic content (TPC), readily bioavailable minerals, soluble fibers, flavonoids, amount of probiotics and prebiotics and antioxidant activity.

The protein-rich biomass described above may be used for human and/or animal consumption without further processing or modifications. Alternatively, or additionally, the protein-rich biomass may be used in a food product for human and/or animal consumption. In particular, the protein-rich biomass may be processed such that it is developed into a product resembling meat, e.g. by means of heat treatment.

The examples/aspects/embodiments related to a method for manufacturing a food product resembling meat from the protein-rich biomass manufactured by the method described above are not according to the invention and are included herein for illustration purposes only.

Disclosed, but not claimed is a method for manufacturing a food product resembling meat, the method comprising the step of:
j) providing a protein-rich biomass comprising at least one fermented substrate and at least one strain of an edible filamentous fungus.

The method for manufacturing a food product resembling meat further comprises at least one of the steps:
k) heat treating the protein-rich biomass via dry heating, pressure heating and steam heating in temperatures between 40°C to 150°C, pressure of up to 5 bar, water content up to 50%;
l) extruding the protein-rich biomass.

As mentioned above, a textured and highly functional edible protein-rich biomass is obtained by the method by fermenting the above mentioned substrates individually or in combination together with an edible fungus or combination of fungi that do not form fruiting bodies from *Ascomycota phylum* and/or *Zygomycota* (particularly *Rhizopus* spp., *Aspergillus* spp., *Neurospora* spp., *Monascus* spp. and *Rhizomucor* spp.). The obtained biomass is densely packed with edible mycelia of particular fungi which cascades intricately into the substrate and hence provides a well textured and resilient structure which closely resembles meat. The obtained functional biomass may be chopped and dried at 40-80°C, more preferably between 55-70°C for 10-90 minutes to achieve brown meat like chunks which may be rehydrated to be developed into a number of meat analogues by mixing it with additives, seasoning, variety of spices and varied heat treatment over a period of time.

In order to enhance the texture of the protein-rich biomass such that it resembles meat, non-claimed step k) of heat treating the protein-rich biomass may be performed. Heat treatment according to step k) may be performed at a temperature range from 40 to 150 °C pressure of up to 5 bar and water content up to 50% for 5-45 min in a plurality of cycles applying different temperature and pressure and time intervals in each cycle. The number of cycles may be two, three or four.

In order to enhance the texture of the protein-rich biomass, non-claimed step I) may be performed, preferably by the use of an extruder e.g. twin-screw co-rotating system. The protein-rich biomass may be extruded between 60-120 °C in a continuous process, which introduces the texture into the biomass by affecting the tertiary structure of the protein component of the biomass. The extrusion process according to step I) specifically enhances the qualities like tensile strength, integrity index and layer structure of the biomass fibers. This in turn enhances the chewiness of the food product leading to its resemblance with a conventional meat product.

It should be noted that the non-claimed method for manufacturing a food product resembling meat may comprise step k), step I) or both step k) and I). Further, the method for manufacturing a food product resembling meat may comprise additional steps of adding supplementary ingredients such as binders or seasoning.

Disclosed, but not claimed is a food and/or feed product comprising the protein-rich biomass of the present invention, which may be applied as meat analogues or products that do not imitate or substitute meat. Such could be but not limited to pellets, flakes, a burger patty, a non-meat ball, a nugget, a sausage, a fritter, as well as baked products such as a cookie, bread, or the like. The food product may further comprise a conventional food additive, such as seasoning, butter, egg, milk, or the like.

The examples/aspects/embodiments related to a system for manufacturing a protein-rich biomass are not according to the invention and are included herein for illustration purposes only.

Disclosed, but not claimed is a system for manufacturing a protein-rich biomass comprising at least one fermented substrate and at least one strain of an edible filamentous fungus. The system comprises at least one fermentation reactor arranged for receiving at least one substrate to be fermented and at least one strain of an edible filamentous fungus. The fermentation reactor may have any suitable shape and size, depending on the desired production capacity and space available for the fermentation reactor. The term "reactor" encompasses a closed space such as a chamber or a room.

Disclosed, but not claimed is the at least one fermentation reactor, which comprises at least one sensor being selected from a group comprising a gas sensor, a temperature sensor, and a humidity sensor. The temperature sensor incorporated in the fermentation reactor may have a detection range of -70°C to 300°C. Preferably, the fermentation reactor comprises at least one gas sensor, at least one temperature sensor, and at least one humidity sensor. The at least one gas sensor may be a CO₂ sensor, an O₂ sensor, a NH₃ sensor or a N₂ sensor. The CO₂ sensor incorporated in the fermentation reactor may have a detection range from 500 to 50000 ppm. In particular, the fermentation reactor may comprise four gas sensors, each detecting one of the gases mentioned above. Each of the gas sensor, the temperature sensor and the humidity sensor may communicate with an operator or with a control system as will be described in greater detail below.

Disclosed, but not claimed is the at least one fermentation reactor, which further comprises at least one fermentation surface, on which the fermentation process of the inoculated substrate occurs. The fermentation surface may be a tray, a container, a plate, or any type of box. The fermentation surface preferably comprises a bottom portion extending horizontally, and elevated edge portions extending vertically. The area of the bottom portion may be from 0.5 m² to 3 m². The vertical extension of the elevated edge portion may be from 0.5 cm to 50 cm. The shape of the bottom portion of the at least one fermentation surface may be circular, triangular, rectangular, or polygonal, and depends on the shape and size of the fermentation reactor.

Preferably, the non-claimed fermentation reactor comprises a plurality of fermentation surfaces. By the term "plurality" in the context of the present invention is understood at least two. The plurality of the fermentation surfaces is preferably arranged horizontally and in parallel to each other in a stacked manner. Alternatively, the plurality of the fermentation surfaces may be arranged vertically. The distance between each two fermentation surfaces may be from 5 to 25 mm. Each fermentation surface of the plurality of the fermentation surfaces may have the shape being same as or different from the shape of the other fermentation surfaces.

Disclosed, but not claimed is the fermentation reactor which further comprises at least one duct arranged in proximity of the at least one fermentation surface, wherein the at least one duct is arranged for supplying at least one of at least one second gaseous fluid, heat, and water vapor into the fermentation reactor. As mentioned above, the at least one second gaseous fluid may be air, oxygen (O₂) or nitrogen (N₂). Preferably, the at least one duct is arranged above the at least one fermentation surface, such that a good contact is obtained between the medium supplied through the at least one duct and the inoculated substrate arranged on the at least one fermentation surface. If a plurality of fermentation surfaces is present, the fermentation reactor preferably comprises a plurality of ducts, wherein at least one duct is assigned to each fermentation surface. The size and shape of the cross-section of the duct depends on the size of the fermentation surface and should be designed such that a sufficient amount of air, heat, water vapor or combination thereof is supplied to the fermentation surface in order to achieve optimal propagation of the at least one edible filamentous fungus.

As mentioned above, it may be desired to minimize human interaction with the fermentation reactor in order to avoid contamination. To this end, disclosed, but not claimed is the system, which may further comprise a robot-assisted loading device arranged upstream of the at least one fermentation reactor and/or a robot-assisted unloading device arranged downstream of the at least one fermentation reactor. The robot-assisted loading device may be arranged for performing at least one of steps a), b) and c) of the method described above. In particular, the robot-assisted loading device may be arranged for loading the at least one substrate to be fermented on the at least one fermentation surface, and for adding the at least one of the edible filamentous fungus to the substrate. The robot-assisted unloading device may be arranged for unloading the protein-rich biomass upon completion of step d), step f) or step g) above.

Preferably, the non-claimed system further comprises a control system being in communication with the at least one sensor, wherein the control system is arranged to control supply of the at least one of at least one second gaseous fluid, heat or water vapor into the fermentation reactor via the at least one duct in response to an input provided by the at least one sensor. According to such an embodiment, an optimal growth of the at least one edible filamentous fungus is obtained in all the stages of the propagation. In particular, when a CO₂ sensor indicates levels in the range of 20000-30000 ppm, confirming an active growth stage, the control system automatically increases the air flow rate, as described above. The light intensity is set at 4.8·10¹⁴ Hz.

It should be understood that automation and the robotic system integrated in the non-claimed fermentation reactor are based on artificial intelligence that detects the propagation of fungal growth. This implies that the robotic arms are smartly synchronized with the sensors that monitor and control the fermentation process and thus control the key parameters such as humidity, temperature, air flow and light intensity. It implies that the ready biomass can be automatically retrieved from the reactor once the desired qualities are achieved, leaving the rest in the same cycle to proceed with the fermentation process until the desired characteristics are reached.

It may be necessary to sterilize the non-claimed fermentation reactor after unloading a batch of the protein-rich biomass. Sterilization of the fermentation device may be done in several cycles by performing the following steps:
- ramping up the temperature to 140-300°C at a rate of between 10-20°C per minute;
- maintaining the temperature at 140-300°C for 30-45 minutes;
- ramping down the temperature at a rate of between 10-20°C per minute.

It should be noted that steps d), g) and f) may all be performed in the non-claimed fermentation reactor.

In order to increase production capacity of the non-claimed system, the system may comprise a plurality of fermentation reactors, and a control unit arranged to independently monitor and control each fermentation reactor within the plurality of fermentation reactors. By operating a plurality of fermentation reactors in parallel separate disinfection procedures can be performed in the fermentation reactors that has been detected to have contamination without affecting an entire batch.

### BRIEF DESCRIPTION OF THE DRAWINGS

Embodiments of the invention will now be described by way of example with reference to the accompanying drawings, of which:
Fig. 1 is a flow chart of the method according to the preset invention;
Fig. 2 is a schematic illustration of the non-claimed system for manufacturing a protein-rich biomass.

### DETAILED DESCRIPTION OF THE INVENTION

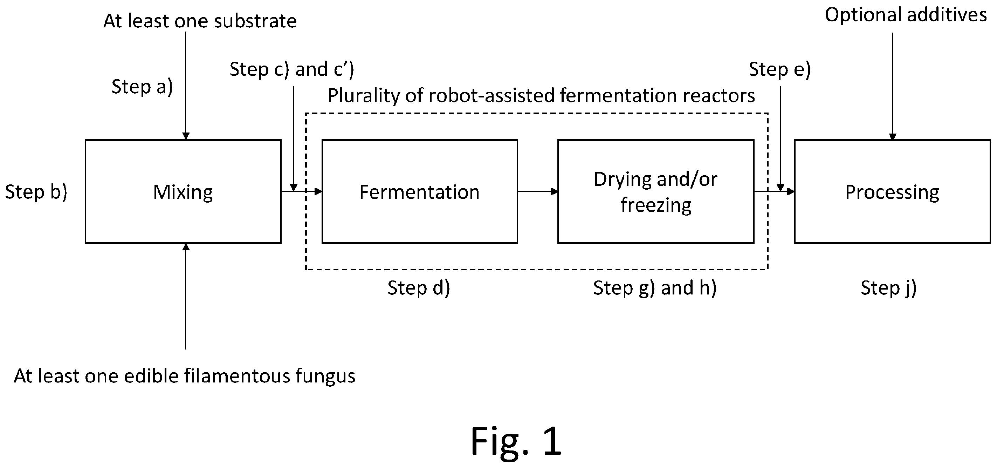

The method for manufacturing a protein-rich biomass comprising at least one fermented substrate and at least one strain of an edible filamentous fungus according to the present invention is depicted in Fig. 1. As may be seen, the method comprises the steps of:
a) providing at least one substrate to be fermented, the substrate(s) having total solid loading of from 51% to 85%; wherein said at least one substrate is selected from a group consisting of root vegetables and residues and derivatives thereof, grains and residues and derivatives thereof, legumes and residues and derivatives thereof, marine organisms and residues and derivatives thereof, potato protein liquor (PPL), distillers dried grain with solubles (DDGS) as well as precursors and residue thereof, and mixtures thereof;
b) inoculating the at least one substrate with at least one strain of an edible filamentous fungus thus obtaining at least one inoculated substrate;
c) setting a set of conditions, wherein the set of conditions comprises at least one of humidity level of from 20 to 100%, flow rate of a second gaseous fluid i.e. air, oxygen or nitrogen of from 0.25 to 4 vvm, temperature of from 30°C to 45°C, light intensity of from 4·10¹⁴ Hz to 5·10¹⁴ Hz and pH of from 3 to 7;
d) fermenting the at least one inoculated substrate at the set of conditions while continuously monitoring at least one parameter, thus obtaining a protein-rich biomass comprising at least one fermented substrate and at least one strain of an edible filamentous fungus;

wherein said at least one strain of an edible filamentous fungus is selected from a group consisting of *Rhizopus* spp., *Aspergillus* spp., *Neurospora* spp., *Monascus* spp. and *Rhizomucor* spp., and
wherein the set of conditions is amended as a function of the at least one parameter.

As mentioned above, during step d), the inoculated substrate is fermented starting with the set of conditions obtained in step c) while continuously monitoring at least one parameter, thus obtaining a protein-rich biomass comprising at least one fermented substrate and at least one strain of an edible filamentous fungus. The at least one parameter mentioned above will change depending on the propagation phase of the at least one edible filamentous fungus. According to the present invention, the set of conditions is amended as a function of the at least one parameter. In other words, the method of the present invention is adapted to the propagation phase of the at least one edible filamentous fungus.

In order to avoid contamination of the substrate to be fermented, human contact should be minimized, especially during step d). To this end, the method of the present invention depicted in Fig. 1 comprises step c') of robot-assisted loading the at least one inoculated substrate into a fermentation chamber and step e) of robot-assisted unloading of the protein-rich biomass from the fermentation chamber. By introduction of these steps, human-induced contamination is greatly reduced or even eliminated since the robots may be exclusively remotely controlled without interaction with the fermentation area.

In order to preserve the protein-rich biomass for a prolonged period of time, for instance for transportation, or to prepare the protein-rich biomass for a further processing, the method depicted in Fig. 1 further comprises step g) of freezing the protein-rich biomass and step h) of drying the protein rich biomass.

As mentioned above, the protein-rich biomass according to the present invention may be used for human and/or animal consumption without further processing or modifications. Alternatively, or additionally, the protein-rich biomass may be used in a food product for human and/or animal consumption, as depicted in Fig. 1 and as has been described above.

The method of the present invention will now be described by examples.

### Example 1

### Steps a") and a)

DDGS is soaked in cold water for 20-120 minutes. Once dissolved, draining is performed such that only the absorbed water is present in the DDGS. The substrate should be slightly moist and crumply. Due to remaining moisture, no additional water needs to be added.

### Steps b), b'), c) and c')

The substrate is spread onto a fermentation surface, acidic or alkaline agents are added prior to inoculation to adjust the pH value. First half of the edible filamentous fungus is evenly stirred into the substrate, the second half is evenly sprinkled on top. The ratio between the edible filamentous fungus and the substrate is approximately 1:40.

### Steps d), f) and g)

The temperature is set to 35°C, more preferably around 35 °C, pH of fermentation medium is set to 4.8, humidity is set to 85% Rh and air flow rate set at a minimum of 0.25 vvm. The airflow is adjusted automatically based on the CO₂ concentration detected in the fermentation reactor. When the CO₂ levels are in the range of 20000-30000 ppm, the installed sensors spontaneously attune the air flow rate to between 0.5-4 vvm. The substrate is fermented for at least 24 hours and more preferably for 40 to 50 hours.

The protein-rich biomass is heat-treated at 65 °C for 20-30 minutes in the above described fermentation reactor. The RNA degrades into monomers and diffuses out of cells leaving the RNA content of fungus well below 2% by weight. The thus obtained protein-rich biomass is subsequently frozen in the fermentation reactor. The freezing cycles are performed by reducing the temperature down to -50 to -40°C for up to 1 hour. The texture of the protein-rich biomass may be enhanced by using an extrusion device prior to preparing the desirable food product.

### Example 2

### Steps a") and a)

The root vegetables are pretreated with heat (e.g. boiling or steaming) for 30 minutes after appropriate size reduction. The root vegetables are then allowed to steam off and cool down to room temperature. Once they are cooled down, they are chopped into pieces. The second, grain-based, substrate does not require pretreatment. The root vegetables and the grain-based substrate are mixed. Due to the moisture remaining in the root vegetables, no additional water will be added.

### Steps b), b'), c) and c')

The substrate is spread onto a fermentation surface, acidic or alkaline agents are added prior to inoculation to adjust the pH value. First half of the edible filamentous fungus is evenly stirred into the substrate, the second half is evenly sprinkled on top. The ratio between the edible filamentous fungus and the substrate is approximately 1:40.

### Steps d), f) and g)

The temperature is set at between 30-40°C, more preferably around 35 °C, pH of fermentation medium is set to 5, humidity is set to 85% Rh and air flow rate set at a minimum of 0.25 vvm. The airflow is adjusted automatically based on the CO₂ concentration detected in the fermentation reactor. When the CO₂ levels are in the range of 20000-30000 ppm, the installed sensors spontaneously attune the air flow rate to between 0.5-4 vvm. The substrate is fermented for at least 48 hours.

The protein-rich biomass is heat-treated at 65 °C for 20-30 minutes in the above-described fermentation reactor. The RNA degrades into monomers and diffuses out of cells leaving the RNA content of fungus well below 2% by weight. The thus is subsequently frozen in the fermentation reactor. The freezing cycles are performed by reducing the temperature down to -50 to -40°C for up to 1 hour.

### Characterization of the protein-rich biomass obtained by the method of the present invention

The protein-rich biomass of the present invention consists of densely grown and consistently cascading mycelium. The protein content of the fermented product lies between 10-60% crude protein. The details are presented in Table 1.

**Table 1**

| **Contents** | | **Example** | | | |
|---|---|---|---|---|---|
| | | 1 | 2 | 3 | 4 |
| Substrate | | Potatoes | Oat bran and beetroot (1:1) | Rolled oats | DDGS |
| Edible filamentous fungus | | *A. oryzae* | *A. oryzae* | *R.oligosporus* | *R.oligospporus* |
| L-carnitine (mg/l) | | 0.8 | 1.3 | 1.6 | 2.5 |
| β-glucan (w/w %) | | - | 4.3 | 5 | - |
| Dietary fiber (w/w %) | | | | 13 | 7 |

| | Unit | µmol/g of fermented product (DWB) | % DWB of fermented product | % DWB of fermented product | g/kg crude protein |
|---|---|---|---|---|---|
| Amino acid | Thr | 30.3 | 0.22 | 0.58 | 40.1 |
| | Leu | 38.1 | 0.58 | 0.97 | 119.8 |
| | Phe | 24.5 | 0.46 | 0.76 | 50.6 |
| | Lys | 33.5 | 0.37 | 0.61 | 38.5 |
| | Arg | 31.6 | 0.52 | 0.89 | 51.0 |
| | His | 13.1 | 0.21 | 0.32 | 41.9 |
| | Ile | 26.2 | 0.31 | 0.51 | 41.7 |
| | Val | 46.3 | 0.40 | 0.69 | 61.8 |
| | Met | | 0.17 | 0.30 | 25.8 |
| Free fatty acid (% of extracted lipid fraction) | Palmitic | | 12 | 13.7 | |
| | Stearic | | 0.8 | 1.5 | |
| | Oleic | | 43.5 | 42 | |
| | Linoleic | | 39 | 40.5 | |
| | Linolenic | | 0.7 | 0.5 | |

### Non-claimed, illustrative method for manufacturing food products comprising protein-rich biomass

As mentioned above, a textured and highly functional edible protein-rich biomass is obtained by the method of the present invention by fermenting the above mentioned substrates individually or in combination together with an edible fungus or combination of fungi from *Ascomycota phylum* and/or *Zygomycota.* The obtained biomass is densely packed with edible mycelia of particular fungi which cascades intricately into the substrate and hence provides a well textured and resilient structure which closely resembling meat. The obtained functional biomass was chopped and dried at 60°C for 65 minutes to achieve brown meat like chunks which may be rehydrated to be developed into a number of meat analogues by mixing it with additives, seasoning, variety of spices and varied heat treatment over a period of time.

### Example 3

### Ingredients

100 g fresh raw protein-rich biomass

### 1 tablespoon margarin

Seasoning will be added in differing amounts and may include the following ingredients: salt, onion, garlic, yellow onion, spring onion, egg or the vegan replacement, soy sauce, black pepper, basil, binder(s) such as methyl cellulose, potato starch gel, kappa carrageenan, sodium alginate and combination thereof.

### Method

- Marinate the biomass by soaking in savory marinate (salt, sugar, soy sauce, monosodium glutamate, vegetable broth powder), remove the excess liquid by filtering
- Heat the marinated biomass in temperature range of 60 to 120 °C for 5-45 min in 4 cycles applying different temperatures and time intervals in each cycle
- Cool down the heat-treated biomass, ground roughly, coat with one of the above mentioned binders or their combination, remove the excess binder
- Heat for 5-45 minutes at temperature range of 50-100°C
- Sauté minced garlic and onion in margarin and add it to the biomass
- Add spices and further binders if needed
- Add egg or egg replacement
- Form the obtained mass into a meat analogue, e.g. a hamburger patty, heat for 10-60 mins, cool down and freeze
- Fry the meat analogue like a regular meat product

### Example 4

### Ingredients

250 g fresh biomass
150 mL milk
2 eggs or corresponding amount of vegan replacement

Seasoning will be added in differing amounts and may include the following ingredients: salt, pepper white and black, brown sugar, white sugar, roasted onion, garlic, ginger, nutmeg, turmeric, binder(s) such as corn starch, potato starch gel, methyl cellulose and combinations thereof.

### Method

- Mix all the ingredients together
- Treat the mixture by three heat-cool cycles, wherein the heating phase in each cycle is 5, 10 and 15 minutes, respectively
- Pour the mixture into a baking form and cut into bites
- Dip each bite into whipped egg white or vegan replacement
- Dip each bite into bread crumb, cornflakes crumb or the like
- Deep fry at medium heat

### Example 5

### Ingredients

180 g fresh fermented biomass dried between 50-80°C for 10-50 minutes, cooled down and powdered
100 g sugar in equal amount of brown and white sugar
1 teaspoon salt
120 g butter added with milk
vanilla sugar
all purpose flour
baking powder
baking soda
raw ginger powder
mixture of nuts
mixture of dried fruits
cardamom
sesame seeds
chocolate chips
fresh cream

### Method

- Whisk together sugar, brown sugar, salt, and butter until combined
- Whisk in milk and vanilla sugar until all sugar has dissolved and the butter is smooth
- Sift in the biomass with the remaining ingredients
- Fold the mixture with a spatula and chill the dough for 10-60 min
- Scoop the dough with a spoon, form round balls with hands, and place them on parchment paper lined baking sheet
- Bake for 10-15 minutes till golden brown in preheated oven to 180°C

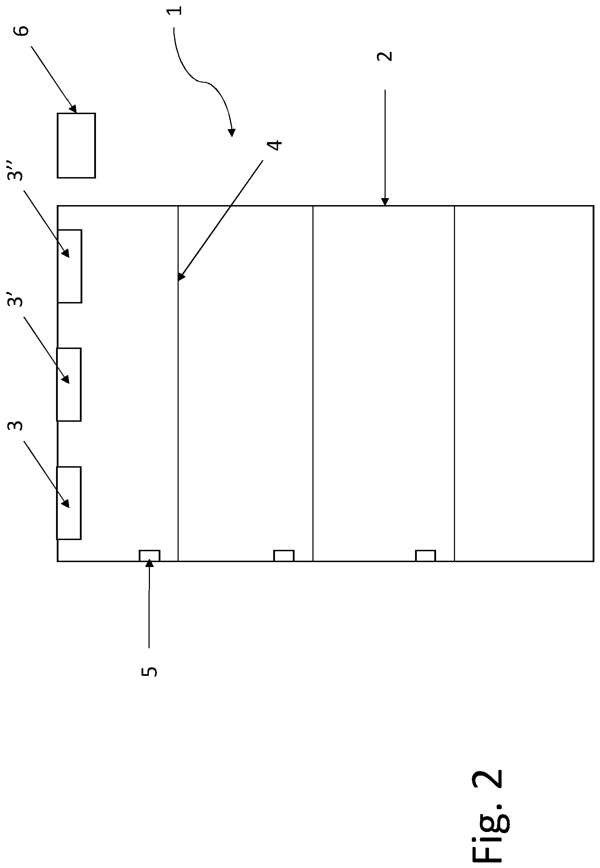

Fig. 2 illustrates a non-claimed system 1 for manufacturing a protein-rich biomass comprising at least one fermented substrate and at least one strain of an edible filamentous fungus. The system 1 comprises a fermentation reactor 2 arranged for receiving at least one substrate to be fermented and at least one strain of an edible filamentous fungus. The fermentation reactor 2 depicted in Fig. 2 has a rectangular cross-section.

The non-claimed fermentation reactor 2 comprises three sensors 3, 3' and 3" being a gas sensor, a temperature sensor, and a humidity sensor.

The non-claimed fermentation reactor 2 further comprises three fermentation surfaces 4, on which the fermentation process of the inoculated substrate occurs. The fermentation surfaces 4 are arranged horizontally and in parallel to each other in a stacked manner.

The non-claimed fermentation reactor 2 further comprises three ducts 5 arranged in proximity of each of the fermentation surfaces 4, wherein the ducts 5 are arranged for supplying at least one of at least one second gaseous fluid, heat, and water vapor into the fermentation reactor. The at least one second gaseous fluid may be air, oxygen (O₂) or nitrogen (N₂). As may be seen in Fig. 2, the ducts 5 are arranged above the fermentation surfaces 4, such that a good contact is obtained between the medium supplied through the ducts 5 and the inoculated substrate arranged on the fermentation surfaces 4.

The non-claimed system 1 according to the present invention further comprises a control system 6 being in communication with the sensors 3, 3' and 3", wherein the control system is arranged to control supply of the at least one of at least one second gaseous fluid, heat or water vapor into the fermentation reactor via the ducts 5 in response to an input provided by the sensors 3, 3' and 3".

## Claims

1. A method for manufacturing a protein-rich biomass comprising at least one fermented substrate and at least one strain of an edible filamentous fungus, said method comprising the steps of:
a) providing at least one substrate to be fermented, said substrate(s) having total solid loading of from 51% to 85%; wherein said at least one substrate is selected from a group consisting of root vegetables and residues and derivatives thereof, grains and residues and derivatives thereof, legumes and residues and derivatives thereof, marine organisms and residues and derivatives thereof, potato protein liquor (PPL), distillers dried grain with solubles (DDGS) as well as precursors and residue thereof, and mixtures thereof;
b) inoculating said at least one substrate with at least one strain of an edible filamentous fungus thus obtaining at least one inoculated substrate;
c) setting a set of conditions, wherein said set of conditions comprises humidity level of from 20% to 100%, flow rate of a second gaseous fluid of from 0.25 to 4 vvm, temperature of from 30°C to 45°C, light intensity of from 4.0 x 10¹⁴ to 5.0 x 10¹⁴ Hz and pH of from 3 to 7;
d) fermenting said at least one inoculated substrate at said set of conditions while continuously monitoring at least one parameter, thus obtaining a protein-rich biomass comprising at least one fermented substrate and at least one strain of an edible filamentous fungus;
wherein said at least one strain of an edible filamentous fungus is selected from a group consisting of *Rhizopus* spp., *Aspergillus* spp., *Neurospora* spp., *Monascus* spp. and *Rhizomucor* spp., and
wherein said set of conditions is amended as a function of said at least one parameter.

2. The method according to claim 1, wherein said method further comprises step c') of robot-assisted loading of said at least one inoculated substrate into a fermentation chamber and/or step e) of robot-assisted unloading of said protein-rich biomass from said fermentation chamber.

3. The method according to claim 1 or 2, wherein said method comprises step a') of adding at least one binder, wherein said step a') occurs simultaneously with step a).

4. The method according to any one of the preceding claims, wherein said method further comprises step f) of heat treatment of said protein-rich biomass, wherein step f) occurs immediately after step d).

5. The method according to any one of the preceding claims, wherein said method further comprises step g) of freezing said protein-rich biomass and/or step h) of drying said protein-rich biomass.

## Patentansprüche

1. Verfahren zur Herstellung einer proteinreichen Biomasse, die wenigstens ein fermentiertes Substrat und wenigstens einen Stamm eines essbaren Fadenpilzes umfasst, wobei das Verfahren die Schritte umfasst:
a) Bereitstellen wenigstens eines Substrats, das fermentiert werden soll, wobei das/die Substrat(e) eine Gesamtfeststoffbeladung von 51 % bis 85 % aufweisen; wobei das wenigstens eine Substrat ausgewählt ist aus einer Gruppe bestehend aus Wurzelgemüse und Rückständen und Derivaten davon, Getreide und Rückständen und Derivaten davon, Hülsenfrüchten und Rückständen und Derivaten davon, Meeresorganismen und Rückständen und Derivaten davon, Kartoffelproteinflüssigkeit (PPL), getrockneter Getreideschlempe mit löslichen Stoffen (DDDGS) sowie Vorläufern und Rückständen davon und Gemischen davon;
b) Inokulieren des wenigstens einen Substrats mit wenigstens einem Stamm eines essbaren Fadenpilzes, um wenigstens ein inokuliertes Substrat zu erhalten;
c) Einstellen eines Satzes von Bedingungen, wobei der Satz von Bedingungen ein Feuchtigkeitsniveau von 20 % bis 100 %, eine Durchflussrate eines zweiten gasförmigen Fluids von 0,25 bis 4 vvm, eine Temperatur von 30 °C bis 45 °C, eine Lichtintensität von 4,0 x 10¹⁴ bis 5,0 x 10¹⁴ Hz und einen pH-Wert von 3 bis 7 umfasst;
d) Fermentieren des wenigstens einen inokulierten Substrats bei dem Satz von Bedingungen, wobei wenigstens ein Parameter kontinuierlich überwacht wird, um eine proteinreiche Biomasse zu erhalten, die wenigstens ein fermentiertes Substrat und wenigstens einen Stamm eines essbaren Fadenpilzes umfasst;
wobei der wenigstens eine Stamm eines essbaren Fadenpilzes ausgewählt ist aus einer Gruppe bestehend aus *Rhizopus* spp., *Aspergillus* spp., *Neurospora* spp., *Monascus* spp. und *Rhizomucor* spp., und
wobei der Satz von Bedingungen als Funktion des wenigstens einen Parameters abgeändert wird.

2. Verfahren nach Anspruch 1, wobei das Verfahren ferner Schritt c') des roboterunterstützten Ladens des wenigstens einen inokulierten Substrats in eine Fermentationskammer und/oder Schritt e) des roboterunterstützten Entladens der proteinreichen Biomasse aus der Fermentationskammer umfasst.

3. Verfahren nach Anspruch 1 oder 2, wobei das Verfahren Schritt a') des Zugebens wenigstens eines Bindemittels umfasst, wobei der Schritt a') gleichzeitig mit Schritt a) erfolgt.

4. Verfahren nach einem der vorstehenden Ansprüche, wobei das Verfahren ferner Schritt f) von Wärmebehandlung der proteinreichen Biomasse umfasst, wobei Schritt f) unmittelbar nach Schritt d) erfolgt.

5. Verfahren nach einem der vorstehenden Ansprüche, wobei das Verfahren ferner Schritt g) des Gefrierens der proteinreichen Biomasse und/oder Schritt h) des Trocknens der proteinreichen Biomasse umfasst.

## Revendications

1. Procédé de fabrication d'une biomasse riche en protéines comprenant au moins un substrat fermenté et au moins une souche d'un champignon filamenteux comestible, ledit procédé comprenant les étapes de :
a) fourniture d'au moins un substrat à fermenter, ledit ou lesdits substrats ayant une charge totale en solides de 51 % à 85 % ; dans lequel ledit ou lesdits substrats sont choisis dans un groupe constitué par des légumes racines et des résidus et dérivés de ceux-ci, des graines et des résidus et dérivés de celles-ci, des légumineuses et des résidus et dérivés de celles-ci, des organismes marins et des résidus et dérivés de ceux-ci, une liqueur protéique de pomme de terre (PPL), des drêches de distillerie avec solubles (DDGS) ainsi que des précurseurs et résidus de ceux-ci, et des mélanges de ceux-ci ;
b) inoculation dudit au moins un substrat avec au moins une souche d'un champignon filamenteux comestible, obtenant ainsi au moins un substrat inoculé ;
c) établissement d'un ensemble de conditions, dans lequel ledit ensemble de conditions comprend un taux d'humidité de 20 % à 100 %, un débit d'un second fluide gazeux de 0,25 à 4 vvm, une température de 30 °C à 45 °C, une intensité lumineuse de 4,0 x 10¹⁴ à 5,0 x 10¹⁴ Hz et un pH de 3 à 7 ;
d) fermentation dudit au moins un substrat inoculé dans ledit ensemble de conditions tout en surveillant continuellement au moins un paramètre, obtenant ainsi une biomasse riche en protéines comprenant au moins un substrat fermenté et au moins une souche d'un champignon filamenteux comestible ;
dans lequel ladite au moins une souche d'un champignon filamenteux comestible est choisie dans un groupe constitué par *Rhizopus* spp., *Aspergillus* spp., *Neurospora* spp., *Monascus* spp. et *Rhizomucor* spp., et
dans lequel ledit ensemble de conditions est modifié en fonction dudit au moins un paramètre.

2. Procédé selon la revendication 1, dans lequel ledit procédé comprend en outre l'étape c') de chargement assisté par robot dudit au moins un substrat inoculé dans un compartiment de fermentation et/ou l'étape e) de déchargement assisté par robot de ladite biomasse riche en protéines dudit compartiment de fermentation.

3. Procédé selon la revendication 1 ou 2, dans lequel ledit procédé comprend l'étape a') d'ajout d'au moins un liant, dans lequel ladite étape a') se produit simultanément à l'étape a).

4. Procédé selon l'une quelconque des revendications précédentes, dans lequel ledit procédé comprend en outre l'étape f) de traitement thermique de ladite biomasse riche en protéines, dans lequel l'étape f) a lieu immédiatement après l'étape d).

5. Procédé selon l'une quelconque des revendications précédentes, dans lequel ledit procédé comprend en outre l'étape g) de congélation de ladite biomasse riche en protéines et/ou l'étape h) de séchage de ladite biomasse riche en protéines.
